# EUROPÄISCHE PATENTANMELDUNG

(11) **EP 1 909 093 A2**
(43) Veröffentlichungstag der Anmeldung: **09.04.2008**
(21) Anmeldenummer: 07112660.1
(22) Anmeldetag: 18.07.2007
(51) Int. Cl.: G01N 19/04, G01N 33/34, G01N 33/36

(54) **Beschichtungsprüfung**

(30) Priorität: 06.10.2006 DE 102006047776
(71) Anmelder: Voith Patent GmbH, 89522 Heidenheim (DE)
(72) Erfinder: Winkels, Klaus, 41836, Hückelhoven (DE)
(74) Vertreter: Kunze, Klaus

(57) **Zusammenfassung**

Die Erfindung betrifft ein Verfahren sowie eine Vorrichtung zum Prüfen der Beschichtung (10) einer Papier-, Karton- oder einer anderen Faserstoffbahn (1) in einer Maschine zur Herstellung und/oder Veredlung derselben, in der die Faserstoffbahn (1) nach dem Auftrag und der Trocknung geglättet wird.

Dabei soll der Zustand der Beschichtung (10) derart erfasst werden, dass die Beschichtung (10) der Faserstoffbahn (1) während oder nach der Trocknung, aber vor der Glättung mit wenigstens einer vorzugsweise glatten und mitlaufenden Indikatorfläche (2) in Kontakt gebracht und die Indikatorfläche (2) nach der Wegführung der Faserstoffbahn (1) auf Anhaftungen der Beschichtung (10) geprüft wird.

## Beschreibung

Die Erfindung betrifft ein Verfahren zum Prüfen der Beschichtung einer Papier-, Karton- oder einer anderen Faserstoffbahn in einer Maschine zur Herstellung und/oder Veredlung derselben, in der die Faserstoffbahn nach dem Auftrag und der Trocknung geglättet wird.

Die Erfindung betrifft auch eine entsprechende Vorrichtung zum Prüfen der Beschichtung einer Papier-, Karton- oder einer anderen Faserstoffbahn in einer Maschine zur Herstellung und/oder Veredlung derselben, in der die Faserstoffbahn nach einer Beschichtungsvorrichtung und dem Durchlaufen einer Trocknungsvorrichtung zu einer Glättvorrichtung geführt wird.

Zur Verbesserung der Oberflächeneigenschaften werden Faserstoffbahnen während ihrer Herstellung, d.h. nach ihrer Haupt-Trocknung ein- oder beidseitig mit einer Beschichtung versehen. Diese Beschichtung hat einen relativ hohen Feuchtegehalt und muss nachfolgend auch zur Herausbildung der notwendigen Festigkeit und Haftung an der Faserstoffbahn getrocknet werden.

Im Anschluss wird die Faserstoffbahn dann häufig online durch eine Glättvorrichtung mit einem oder mehreren Glättspalten geführt. Falls die Beschichtung nicht ausreichend getrocknet wurde, kommt es zur Ablösung und Ablagerung von Teilen der Beschichtung auf den Glättwalzen der Glättvorrichtung.

Diese Ablagerungen beeinträchtigen das Glättergebnis wesentlich und können auch zu Schäden am Walzenmantel der Glättwalzen führen, insbesondere wenn diese eine Kunststoffoberfläche aufweisen.

Bekannte Verfahren zur Bestimmung des Feuchtegehaltes der Beschichtung arbeiten nicht genau genug. Außerdem wird insbesondere bei lediglich besonders feuchten Streifen der Faserstoffbahn die Gefahr nicht rechtzeitig erkannt.

Die Aufgabe der Erfindung ist es daher ein genaues und reaktionsschnelles Verfahren sowie eine entsprechende Vorrichtung zur Prüfung der Beschichtung der Faserstoffbahn zu schaffen.

Erfindungsgemäß wurde die Aufgabe hinsichtlich des Verfahrens dadurch gelöst, dass die Beschichtung der Faserstoffbahn während oder nach der Trocknung, aber vor der Glättung mit wenigstens einer vorzugsweise glatten und mit der Faserstoffbahn laufenden Indikatorfläche in Kontakt gebracht und die Indikatorfläche nach der Wegführung der Faserstoffbahn auf Anhaftungen der Beschichtung geprüft wird.

Bei der Vorrichtung ist erfindungswesentlich, dass die Beschichtung der Faserstoffbahn in und/oder nach der Trocknungsvorrichtung, aber vor der Glättvorrichtung mit wenigstens einer vorzugsweise glatten und mitlaufenden Indikatorfläche in Kontakt gebracht wird, wobei der Indikatorfläche nach der Wegführung der Faserstoffbahn eine Prüfvorrichtung zur Erfassung von Anhaftungen der Beschichtung auf der Indikatorfläche zugeordnet ist.

Indem die Beschichtung während oder nach der Trocknung mit der Indikatorfläche in Kontakt kommt, werden die Festigkeit und die Haftung der Beschichtung an der Faserstoffbahn geprüft.

Begünstigt wird dies durch eine glatte Oberfläche der Indikatorfläche, was ein leichtes Anhaften von Teilen der Beschichtung gewährleistet.

Falls die Beschichtung nicht ausreichend getrocknet ist, so lösen sich Teile der Beschichtung entsprechend leichter und bleiben an der Indikatorfläche haften.

Der Grad bzw. Umfang der Anhaftung von Teilen der Beschichtung an der Indikatorfläche ist ein Maß für die Festigkeit und Haftung der Beschichtung an der Faserstoffbahn und damit indirekt auch für den Feuchtegehalt der Beschichtung sowie der Faserstoffbahn.

Dabei ist nämlich zu berücksichtigen, dass die Beschichtung natürlich auch zu einer Befeuchtung der Faserstoffbahn führt, so dass nicht nur eine Trocknung der Beschichtung sondern auch der Faserstoffbahn erforderlich ist.

Übersteigt der Umfang der Anhaftungen von Teilen der Beschichtung an der Indikatorfläche ein bestimmte, vorgebbare Grenze, so sollte die Maschine gestoppt werden.

Da die Faserstoffbahn im Allgemeinen zur Glättung durch wenigstens einen Glättspalt geführt wird, so ist es zum Schutz der Walzenmäntel der entsprechenden Glättwalzen von Vorteil, wenn die Glättspalte geöffnet werden, wenn die Indikatorwalze Anhaftungen aufweist oder diese einen bestimmten Grenzwert überschreiten.

Hierzu sollte die Prüfvorrichtung, welche die Anhaftungen erkennt, mit einer Steuereinheit verbunden sein, die auf die Glättvorrichtung und dort insbesondere auf die Walzentrennung einwirkt.

Vorteile hat die Erfindung insbesondere bei Beschichtungsvorrichtungen, die wenigstens einen Rakel zum Entfernen eines Teiles der Beschichtung unmittelbar nach dem Auftrag besitzen. Bei diesen Beschichtungsvorrichtungen kann die Dicke des Beschichtung erheblich schwanken, wenn der Rakel beispielsweise wegen eines Korns abhebt und so einen geringeren Teil der Beschichtung nach dem Auftrag abrakelt. Im Ergebnis kommt es zu einer ungenügenden Trocknung der Beschichtung mit der Gefahr von Anhaftungen an den Glättwalzen der Glättvorrichtung.

Um die Beschichtung nicht zu beeinträchtigen, sollte die Trocknung der Beschichtung nach dem Auftrag zumindest teilweise kontaktlos erfolgen.

Hierzu sollte die Trocknungsvorrichtung zumindest teilweise von einer kontaktlosen Trocknungsvorrichtung, insbesondere von einem Infrarot-Trockner oder einem Heißluft-Trockner gebildet werden.

Besonderes einfach, genau und schnell wird die Prüfung, wenn die Prüfvorrichtung als optische Prüfvorrichtung, insbesondere als Kamera ausgebildet ist. Die Kamera erfasst dabei die Anhaftungen an der Indikatorfläche auf der Basis von Farbunterschieden zwischen der Farbe der Beschichtung und der Farbe der Indikatorfläche.

Dabei kann diese optische Prüfung der Indikatorfläche auf Anhaftungen noch optimiert werden, wenn die Indikatorfläche eine dunkle Oberfläche aufweist. Dies verstärkt den Kontrast zwischen der dunklen Indikatorfläche und den in der Regel sehr hellen, insbesondere weißen Beschichtungen.

Da die Indikatorfläche während des Kontakts mit der Beschichtung durch Anhaftungen verunreinigt wird, sollte diese nach der Prüfung, aber vor einer Wiederzuführung zur Faserstoffbahn gereinigt werden. Auf diese Weise kann der Zustand der Beschichtung nach einem Kontakt mit der Indikatorfläche sehr genau erfasst werden.

Eine besonders einfache und wirksame Möglichkeit zur Reinigung der Indikatorfläche ergibt sich durch den Einsatz einer Reinigungsvorrichtung, insbesondere in Form einer rotierenden Bürste, welche der Indikatorfläche nach der Prüfvorrichtung zugeordnet sein sollte.

Als Indikatorfläche eignet sich insbesondere ein endlos umlaufendes Band oder eine rotierende Walze, insbesondere in Form einer Leitwalze.

Dabei sollte die Indikatorfläche mindestens 20 m bezogen auf den Bahnweg der Faserstoffbahn vor der Glättung bzw. der Glättvorrichtung, insbesondere dem ersten Glättspalt der Glättvorrichtung angeordnet sein. Vorzugsweise sollte die Faserstoffbahn zwischen 0,5 und 2 s von der Indikatorfläche bis zum ersten Glättspalt benötigen.

Dieser Abstand ermöglicht die rechtzeitige Einleitung entsprechender Maßnahmen nach dem Erkennen eines Fehlers bei der Beschichtung, d.h. insbesondere bevor die Faserstoffbahn mit der fehlerhaften Beschichtung in die Glättvorrichtung gelangt.

Neben einem Stopp der Maschine kann es auch genügen, die Glättspalte der Glättvorrichtung zu öffnen und/oder eine nachfolgende weitere Trocknung zu intensivieren.

Anwendung kann das Verfahren und/oder die Vorrichtung bei einseitig oder auch beidseitig beschichteten Faserstoffbahnen finden.

Bei beidseitig beschichteten Faserstoffbahnen sollten Indikatorfläche auf beiden Seiten der Faserstoffbahn angeordnet werden.

Unabhängig davon kann es ebenso von Vorteil sein, die Faserstoffbahn an mehreren Stellen zwischen der Beschichtung und der Glättung mit einer Indikatorfläche in Kontakt zu bringen.

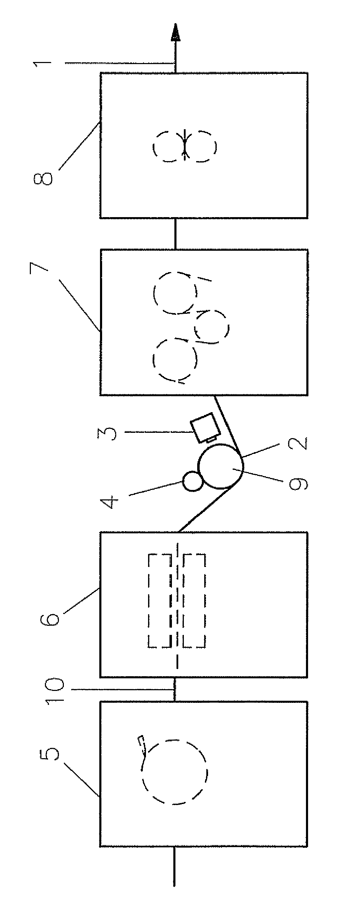

Nachfolgend soll die Erfindung an einem Ausführungsbeispiel näher erläutert werden. In der beigefügten Zeichnung zeigt die Figur einen schematischen Querschnitt durch einen Teil einer Papiermaschine zur Herstellung einer einseitig beschichteten Faserstoffbahn 1, d.h. Papierbahn.

Nach der Haupttrocknung der Faserstoffbahn 1 wird diese in eine Beschichtungsvorrichtung 5 geführt. In dieser erfolgt das Auftragen einer Beschichtung 10, hier beispielhaft auf die Oberseite der Faserstoffbahn 1.

Die Beschichtung 10, auch als Strich bezeichnet, verbessert die Oberflächeneigenschaften der Faserstoffbahn 1 erheblich.

Dabei wird die Beschichtung 10 auf die Faserstoffbahn 1 gesprüht oder mit einer Auftragswalze auf die Faserstoffbahn 1 aufgetragen. Anschließend erfolgt das Abrakeln des überschüssigen Teils der Beschichtung 10 mit einem Rakel, während sich die Faserstoffbahn 1 auf einer Walze abstützt.

Der Rakel erstreckt sich über die gesamte Breite der Faserstoffbahn 1 und soll gewährleisten, dass die Beschichtung 10 eine bestimmte Dicke nicht überschreitet.

Kommt es dabei jedoch zu einer Funktionsstörung, beispielsweise wenn ein Korn den Rakel anhebt, so führt dies zu einer dickeren, auf der Faserstoffbahn 1 verbleibenden Beschichtung 10. Ist dies örtlich begrenzt, so entstehen schmale Streifen mit einer dickeren Beschichtung 10.

Anschließend erfolgt in einer folgenden Trocknungsvorrichtung 6 eine kontaktlose Trocknung der Faserstoffbahn 1, aber insbesondere der Beschichtung 10. Hierzu ist die Trocknungsvorrichtung 6 als Schwebetrockner ausgebildet, welcher die Beschichtung 10 und die Faserstoffbahn 1 mit Heißluft beaufschlagt. Möglich ist jedoch auch der Einsatz von Infrarottrockner.

In einer zweiten, folgenden Trocknungsvorrichtung 7 wird die Faserstoffbahn 1 mäanderförmig über beheizte Trockenzylinder und Leitwalzen geführt.

Nach dem Abschluss der Trocknung gelangt die beschichtete Faserstoffbahn 1 in eine Glättvorrichtung 8.

Diese Glättvorrichtung 8 wird von einem Kalander mit mehreren Glättwalzen gebildet, in dem die Faserstoffbahn 1 mehrere Glättspalten durchläuft.

Falls die Dicke der Beschichtung 10 auch nur örtlich begrenzt eine bestimmte Dicke überschreitet, so führt die Trocknungsbehandlung nicht mehr zur ausreichenden Trocknung der Beschichtung 10. Infolgedessen kann es zur Ablösung von Teilen der Beschichtung 10 in der Glättvorrichtung 8 kommen.

Die gelösten Teile der Beschichtung 10 bleiben dann an den glatten Mantelflächen der Glättwalzen haften. Dies beeinträchtigt die Oberfläche dieser Mantelflächen und damit die Glättung der Faserstoffbahn 1 erheblich und kann sogar zur Beschädigung der Mantelfläche führen.

Um Mängel der Beschichtung 10, insbesondere hinsichtlich Feuchtegehalt, Haftung und Festigkeit feststellen zu können, wird die Beschichtung 10 zwischen den Trocknungsvorrichtungen 6,7 mit einer glatten und mitlaufenden Indikatorfläche 2 in Kontakt gebracht.

In besonders einfacher Ausführung wird die Indikatorfläche 2 hier von einer rotierenden und von der Faserstoffbahn 1 umschlungenen Leitwalze 9 gebildet.

Sind Abschnitte der oder die Beschichtung 10 insgesamt zu feucht, so bleibt ein entsprechend großer Teil der Beschichtung 10 an der glatten Mantelfläche der Leitwalze 9 haften.

Da die Beschichtung 10 weiß ist, kann das Ausmaß der Anhaftungen an der Leitwalze 9 besonders gut erkannt werden, wenn die Mantelfläche der Leitwalze 9 dunkel, insbesondere schwarz ist.

Zur Erfassung der Anhaftungen wird die Mantelfläche der Leitwalze 9 von einer axial entlang der Leitwalze 9 traversierenden Prüfvorrichtung 3 in Form einer Kamera überwacht. Durch eine Farbauswertung kann die Kamera leicht die Anhaftungen erkennen und ein Störsignal zur Maschinensteuerung senden.

Falls die Anhaftungen zu stark sind, d.h. die Beschichtung 10 mangelhaft ist, so bewirkt das Störsignal das Öffnen der Glättspalte der Glättvorrichtung 8, so dass die Glättwalzen nicht verschmutzt werden.

Ist die Beschichtung 10 wieder in Ordnung, können die Glättspalte auch wieder geschlossen werden.

Stattdessen oder ergänzend kann aber auch die Trocknung in den Trocknungsvorrichtungen 6,7, insbesondere in der folgenden 7 intensiviert werden.

Damit für diese Maßnahmen nach einem Störfall genügend Zeit bleibt, ohne eine Verschmutzung der Glättwalzen befürchten zu müssen, ist der Bahnweg der Faserstoffbahn 1 zwischen der Leitwalze 9 und dem ersten Glättspalt größer als 20 m.

Um die Prüfung der Indikatorfläche 2 möglichst genau zu gestalten, wird diese vor dem Kontakt mit der Beschichtung 10 gereinigt. Hierzu ist der Leitwalze 9 in Rotationsrichtung nach der Kamera eine Reinigungsvorrichtung 4 in Form einer rotierenden und axial entlang der Leitwalze 9 traversierenden Reinigungsbürste zuordnet. Dabei hat die Reinigungsbürste die Form einer Walze, deren Rotationsachse achsparallel zur Leitwalze 9 verläuft.

## Patentansprüche

1. Verfahren zum Prüfen der Beschichtung (10) einer Papier-, Karton- oder einer anderen Faserstoffbahn (1) in einer Maschine zur Herstellung und/oder Veredlung derselben, in der die Faserstoffbahn (1) nach dem Auftrag und der Trocknung geglättet wird, **dadurch gekennzeichnet, dass**
die Beschichtung (10) der Faserstoffbahn (1) während oder nach der Trocknung, aber vor der Glättung mit wenigstens einer Indikatorfläche (2) in Kontakt gebracht und die Indikatorfläche (2) nach der Wegführung der Faserstoffbahn (1) auf Anhaftungen der Beschichtung (10) geprüft wird.

2. Verfahren nach Anspruch 1, **dadurch gekennzeichnet, dass**
die Faserstoffbahn mit einer glatten Indikatorfläche (2) in Kontakt gebracht wird.

3. Verfahren nach Anspruch 1 oder 2, **dadurch gekennzeichnet, dass**
die Indikatorfläche (2) mit der Faserstoffbahn (1) mitläuft.

4. Verfahren nach einem der vorhergehenden Ansprüche, **dadurch gekennzeichnet, dass**
ein Teil der Beschichtung (10) unmittelbar nach dem Auftrag abgerakelt wird.

5. Verfahren nach einem der vorhergehenden Ansprüche, **dadurch gekennzeichnet, dass**
die Trocknung der Beschichtung (10) nach dem Auftrag zumindest teilweise kontaktlos erfolgt.

6. Verfahren nach einem der vorhergehenden Ansprüche, **dadurch gekennzeichnet, dass**
die Indikatorfläche (2) optisch auf Anhaftungen geprüft wird.

7. Verfahren nach einem der vorhergehenden Ansprüche, **dadurch gekennzeichnet, dass**
die Faserstoffbahn zur Glättung durch wenigstens einen Glättspalt geführt wird und die Glättspalte geöffnet werden, wenn die Indikatorfläche (2) Anhaftungen aufweist oder diese einen bestimmten Grenzwert überschreiten.

8. Verfahren nach einem der vorhergehenden Ansprüche, **dadurch gekennzeichnet, dass**
die Indikatorfläche (2) nach der Prüfung gereinigt wird.

9. Verfahren nach einem der vorhergehenden Ansprüche, **dadurch gekennzeichnet, dass**
die Indikatorfläche (2) mindestens 20 m (Bahnweg) vor der Glättung angeordnet ist, wobei die Faserstoffbahn (1) vorzugsweise zwischen 0,5 und 2 s von der Indikatorfläche (2) bis zum ersten Glättspalt benötigt.

10. Vorrichtung zum Prüfen der Beschichtung (10) einer Papier-, Karton- oder einer anderen Faserstoffbahn (1) in einer Maschine zur Herstellung und/oder Veredlung derselben, in der die Faserstoffbahn (1) nach einer Beschichtungsvorrichtung (5) und dem Durchlaufen einer Trocknungsvorrichtung (6,7) zu einer Glättvorrichtung (8) geführt wird, insbesondere zur Durchführung des Verfahrens nach einem der vorhergehenden Ansprüche, **dadurch gekennzeichnet, dass**
die Beschichtung (10) der Faserstoffbahn (1) in und/oder nach der Trocknungsvorrichtung (6,7), aber vor der Glättvorrichtung (8) mit wenigstens einer Indikatorfläche (2) in Kontakt gebracht wird, wobei der Indikatorfläche (2) nach der Wegführung der Faserstoffbahn (1) eine Prüfvorrichtung (3) zur Erfassung von Anhaftungen der Beschichtung (10) auf der Indikatorfläche (2) zugeordnet ist.

11. Vorrichtung nach Anspruch 10, **dadurch gekennzeichnet, dass**
die Indikatorfläche (2) glatt ist.

12. Vorrichtung nach Anspruch 11, **dadurch gekennzeichnet, dass**
die Indikatorfläche (2) mitläuft.

13. Vorrichtung nach einem der Ansprüche 10 bis 12, **dadurch gekennzeichnet, dass**
die Beschichtungsvorrichtung (5) wenigstens einen Rakel zum Entfernen eines Teiles der Beschichtung (10) unmittelbar nach dem Auftrag besitzt.

14. Vorrichtung nach einem der Ansprüche 10 oder 13, **dadurch gekennzeichnet, dass**
die Trocknungsvorrichtung (6,7) zumindest teilweise von einer kontaktlosen Trocknungsvorrichtung (6), insbesondere von einem Infrarot-Trockner oder einem Heißluft-Trockner gebildet wird.

15. Vorrichtung nach einem der Ansprüche 10 bis 14, **dadurch gekennzeichnet, dass**
die Prüfvorrichtung (3) von einer optischen Prüfvorrichtung (3), insbesondere einer Kamera gebildet wird.

16. Vorrichtung nach einem der Ansprüche 10 bis 15, **dadurch gekennzeichnet, dass**
die Indikatorfläche (2) eine dunkle Oberfläche aufweist.

17. Vorrichtung nach einem der Ansprüche 10 bis 16, **dadurch gekennzeichnet, dass**
die Indikatorfläche (2) von einer rotierenden Walze, insbesondere von einer Leitwalze (9) gebildet wird.

18. Vorrichtung nach einem der Ansprüche 10 bis 17, **dadurch gekennzeichnet, dass**
der Indikatorfläche (2) nach der Prüfvorrichtung (3) eine Reinigungsvorrichtung (4), insbesondere in Form einer rotierenden Bürste zugeordnet ist.

19. Vorrichtung nach einem der Ansprüche 10 bis 18, **dadurch gekennzeichnet, dass**
die Indikatorfläche (2) mindestens 20 m (Bahnweg) vor der Glättvorrichtung (8) angeordnet ist, wobei die Faserstoffbahn (1) vorzugsweise zwischen 0,5 und 2 s von der Indikatorfläche (2) bis zum ersten Glättspalt benötigt.

20. Vorrichtung nach einem der Ansprüche 10 bis 19, **dadurch gekennzeichnet, dass**
die Prüfvorrichtung (3) mit einer Steuereinheit verbunden ist, welche auf die Glättvorrichtung (8) einwirkt.

21. Anwendung des Verfahrens und/oder der Vorrichtung nach einem der vorhergehenden Ansprüche zur indirekten Erfassung des Feuchtegehaltes der Beschichtung (10).

22. Anwendung des Verfahrens und/oder der Vorrichtung nach einem der vorhergehenden Ansprüche zur Erfassung der Haftung der Beschichtung (10) an der Faserstoffbahn (1).

23. Anwendung des Verfahrens und/oder der Vorrichtung nach einem der vorhergehenden Ansprüche bei einseitig beschichteten Faserstoffbahnen (1).

24. Anwendung des Verfahrens und/oder der Vorrichtung nach einem der Ansprüche 1 bis 22 bei beidseitig beschichteten Faserstoffbahnen (1).
